# EUROPEAN PATENT APPLICATION

(11) **EP 4 328 921 A1**
(43) Date of publication of application: **28.02.2024**
(21) Application number: 21937833.8
(22) Date of filing: 20.04.2021
(51) Int. Cl.: G16B 40/20, G16B 15/00, G16B 50/00

(54) **INFORMATION PROCESSING PROGRAM, INFORMATION PROCESSING METHOD, AND INFORMATION PROCESSING DEVICE**

(71) Applicant: FUJITSU LIMITED, Kawasaki-shi, Kanagawa 211-8588 (JP)
(72) Inventor: KATAOKA, Masahiro, Kawasaki-shi, Kanagawa 211-8588 (JP); WADA, Mitsuhito, Kawasaki-shi, Kanagawa 211-8588 (JP); MATSUMURA, Ryo, Kawasaki-shi, Kanagawa 211-8588 (JP)
(74) Representative: Haseltine Lake Kempner LLP
(86) International application number: PCT/JP2021/015983
(87) International publication number: WO 2022/224336

(57) **Abstract**

An information processing program causes a computer to execute a process including executing training of a trained model on the basis of training data defining relations between vectors corresponding to genomes and vectors respectively corresponding to pluralities of subgenomes composing the genomes and in a case where a genome to be analyzed has been received, calculating vectors of a plurality of subgenomes corresponding to the genome to be analyzed by inputting the genome to be analyzed into the trained model.

## Description

### Technical Field

The present invention relates to information processing programs, for example.

### Background Art

Genetic recombination manipulations have been performed using gene vectors due to advancement of gene transfer technologies and deeper understanding related immunological mechanisms. Mediums with various added characteristics are used as gene vectors differently depending on sizes of gene segments inserted and purposes of insertion. Gene vectors derived from host organisms, such as colon bacilli and yeasts, are used for these manipulations.

For example, chimeric antigen receptor (CAR) introduced T cell therapy has attracted attention as immunotherapy of cancer using genetically modified T cells. A CAR is a receptor that: is artificially made by fusion of a part that specifically recognizes an antigen and that is derived from an antibody with a part derived from a T cell receptor (TCR), the part having a cytotoxic function; specifically recognizes a cancer antigen; and is able to attack the cancer antigen.

### Citation List

### Patent Literature

Patent Literature 1: International Publication Pamphlet No. WO 2020/230240
Patent Literature 2: International Publication Pamphlet No. WO 2007/102578

### Summary

### Technical Problem

Developing gene therapy drugs using gene vectors is very promising but synthesizing gene therapy drugs using various gene vectors, as is, is difficult.

Accordingly, one may consider synthesizing target gene therapy drugs by substitution of various gene vectors, but searching for substitutable gene vectors and efficient genetic modification are actually difficult.

An object of the present invention in one aspect is to provide an information processing program, an information processing method, and an information processing apparatus that enable determination of a genome that serves as a substitute for a subgenome included in a target genome.

### Solution to Problem

According to an aspect of the embodiment of the invention, an information processing program causes a computer to execute a process including executing training of a trained model on the basis of training data defining relations between vectors corresponding to genomes and vectors respectively corresponding to pluralities of subgenomes composing the genomes and in a case where a genome to be analyzed has been received, calculating vectors of a plurality of subgenomes corresponding to the genome to be analyzed by inputting the genome to be analyzed into the trained model.

### Advantageous Effects of Invention

Determination of a genome is enabled, the genome serving as a substitute for a subgenome included in a target genome.

### Brief Description of Drawings

FIG. 1 is a diagram for explanation of a genome.
FIG. 2 is a diagram illustrating relations between: amino acids; and bases and codons.
FIG. 3 is a diagram for explanation of a primary structure, a secondary structure, a tertiary structure, and a higher-order structure of a protein.
FIG. 4 is a diagram illustrating an example of a gene vector.
FIG. 5 is a diagram for explanation of an example of a process in a training phase of an information processing apparatus according to an embodiment.
FIG. 6 is a diagram for explanation of an example of a process in an analysis phase of the information processing apparatus according to the embodiment.
FIG. 7 is a functional block diagram illustrating a configuration of the information processing apparatus according to the first embodiment.
FIG. 8 is a diagram illustrating an example of a data structure of a base file.
FIG. 9 is a diagram illustrating an example of a data structure of a conversion table.
FIG. 10 is a diagram illustrating an example of a data structure of a dictionary table.
FIG. 11 is a diagram illustrating an example of a data structure of a protein primary structure dictionary.
FIG. 12 is a diagram illustrating an example of a data structure of a secondary structure dictionary.
FIG. 13 is a diagram illustrating an example of a data structure of a tertiary structure dictionary.
FIG. 14 is a diagram illustrating an example of a data structure of a higher-order structure dictionary.
FIG. 15 is a diagram illustrating an example of a data structure of a compressed file table.
FIG. 16 is a diagram illustrating an example of a data structure of a vector table.
FIG. 17 is a diagram illustrating an example of a data structure of a protein primary structure vector table.
FIG. 18 is a diagram illustrating an example of a data structure of a secondary structure vector table.
FIG. 19 is a diagram illustrating an example of a data structure of a tertiary structure vector table.
FIG. 20 is a diagram illustrating an example of a data structure of a higher-order structure vector table.
FIG. 21 is a diagram illustrating an example of a data structure of an inverted index table.
FIG. 22 is a diagram illustrating an example of a data structure of a protein primary structure inverted index.
FIG. 23 is a diagram illustrating an example of a data structure of a secondary structure inverted index.
FIG. 24 is a diagram illustrating an example of a data structure of a tertiary structure inverted index.
FIG. 25 is a diagram illustrating an example of a data structure of a higher-order structure inverted index.
FIG. 26 is a diagram illustrating an example of a data structure of a genome dictionary.
FIG. 27 is a first flowchart illustrating a procedure by the information processing apparatus according to the embodiment.
FIG. 28 is a second flowchart illustrating a procedure by the information processing apparatus according to the embodiment.
FIG. 29 is a diagram for explanation of an example of a process in a training phase of an information processing apparatus according to a second embodiment.
FIG. 30 is a diagram for explanation of a process by the information processing apparatus according to the second embodiment.
FIG. 31 is a functional block diagram illustrating a configuration of the information processing apparatus according to the second embodiment.
FIG. 32 is a flowchart illustrating a procedure by the information processing apparatus according to the second embodiment.
FIG. 33 is a diagram illustrating an example of a hardware configuration of a computer that implements functions that are the same as those of the information processing apparatuses according to the embodiments. Description of Embodiments

Embodiments of an information processing program, an information processing method, and an information processing apparatus disclosed in the present application will hereinafter be described in detail on the basis of the drawings. The present invention is not limited by these embodiments.

### First Embodiment

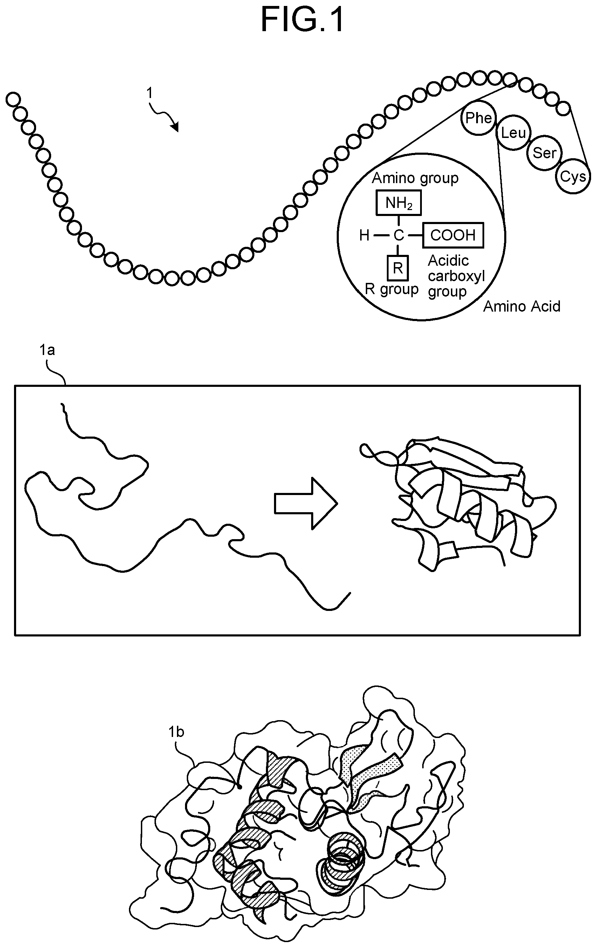

Genomes will be described before description of an embodiment. FIG. 1 is a diagram for explanation of genomes. A genome 1 includes genetic information prescribing a sequence in which plural amino acids are linked to each other. An amino acid is determined by consecutive three bases, that is, a codon. The genome 1 also includes information on a protein 1a. The protein 1a has 20 types of multiple amino acids bonded to each other in a chain. The structure of the protein 1a is able to be considered as a primary structure, a secondary structure, a tertiary structure, or a higher-order (quaternary) structure of proteins. 1b illustrates a higher-order structure of the protein 1a. In the description hereinafter, a primary structure of a protein, a secondary structure of a protein, a tertiary structure of a protein, and a higher-order structure of a protein will respectively be referred to as a primary structure, a secondary structure, a tertiary structure, and a higher-order structure, as appropriate.

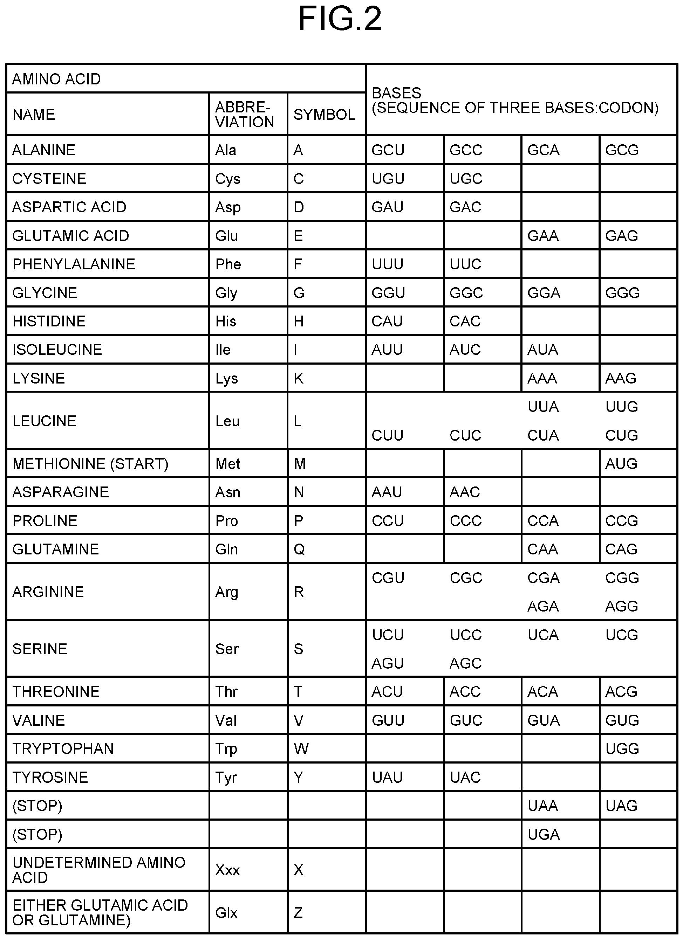

DNAs and RNAs each have four types of bases that are each denoted by a symbol, "A", "G", "C", "T", or "U". Furthermore, sequences each made up of three bases determine 20 types of amino acids. Each of these amino acids is denoted by symbols, "A" to "Y". FIG. 2 is a diagram illustrating relations between: amino acids; and bases and codons. A sequence of three bases is called a "codon". Each sequence of bases determines a codon and an amino acid is determined when a codon is determined.

As illustrated in FIG. 2, plural types of codons are associated with one amino acid. Therefore, determining a codon determines an amino acid, but determining an amino acid does not uniquely determine a codon. For example, the amino acid, "alanine (Ala) A", is associated with a codon, "GCU", "GCC", "GCA", or "GCG".

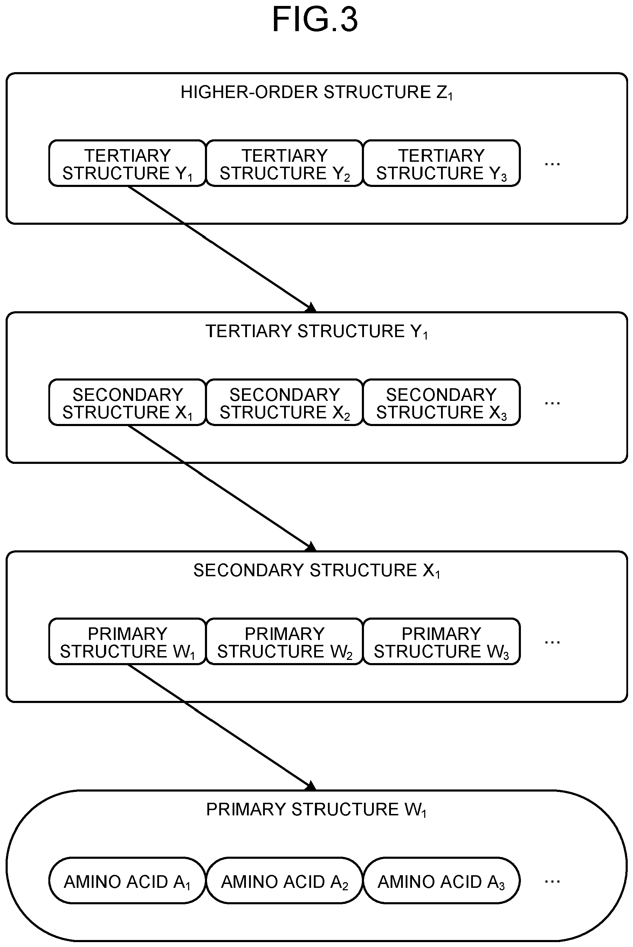

A protein is also uniquely determined by a sequence of bases. A primary structure of a protein is a sequence of plural amino acids. A secondary structure includes α-helixes and β-sheets that are symmetrical substructures observed locally. A tertiary structure includes plural secondary structures. Furthermore, a higher-order structure includes plural tertiary structures. FIG. 3 is a diagram for explanation of a primary structure, a secondary structure, a tertiary structure, and a higher-order structure of a protein. For example, as illustrated in FIG. 3, a higher-order structure Z₁ includes tertiary structures Y₁, Y₂, and Y₃. The tertiary structure Y₁ includes, for example, secondary structures X₂, X₂, and X₃. The secondary structure X₁ includes, for example, primary structures W₂, W₂, and W₃. The primary structure W₁ includes, for example, amino acids A₁, A₂, and A₃.

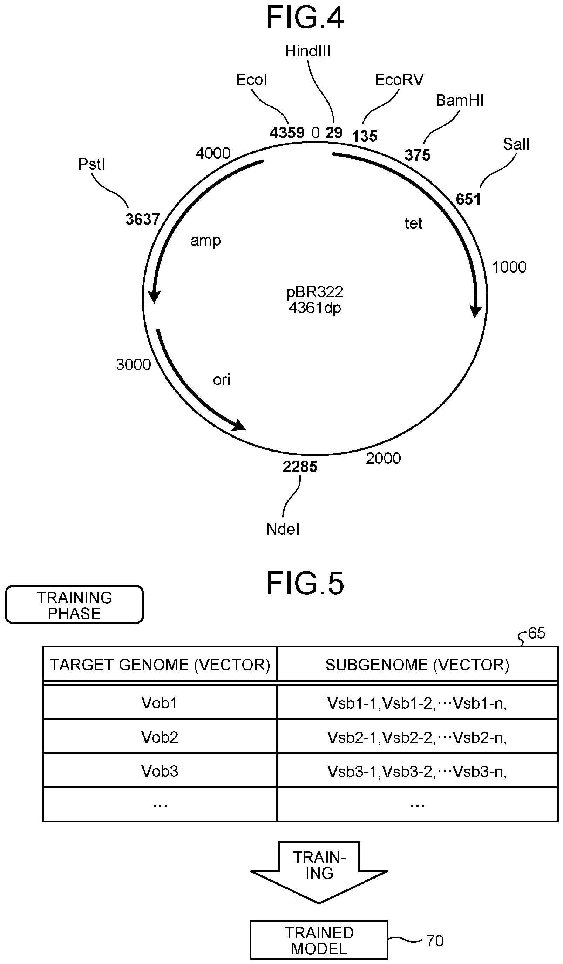

A gene vector used in this embodiment is a DNA or RNA molecule that is used to artificially carry a foreign genetic substance to another cell. Gene vectors include, for example, plasmids, cosmids, lambda phages, and artificial chromosomes. FIG. 4 is a diagram illustrating an example of the gene vector. The gene vector illustrated in FIG. 4 is pBR322 plasmid and is widely used as a cloning vector. Description will be made on the assumption that gene vectors themselves are base sequences of DNAs and RNAs and correspond to, for example, higher-order structures of proteins described by reference to FIG. 3.

Furthermore, the gene vector is generated by synthesis of plural subvectors. Subvectors are base sequences of DNAs and RNAs and correspond to, for example, secondary structures of proteins described by reference to FIG. 3. Subvectors include so-called colon bacillus vectors including elements for maintenance in colon bacilli and vectors for maintenance in cell lines derived from, for example, yeast, plants, and mammals. The subvectors may be other vectors.

An example of a process by an information processing apparatus according to this embodiment will be described next.

FIG. 5 is a diagram for explanation of an example of a process in a training phase of the information processing apparatus according to the embodiment. As illustrated in FIG. 5, the information processing apparatus executes machine training of a trained model 70 by using training data 65. The trained model 70 corresponds to, for example, a convolutional neural network (CNN) or a recurrent neural network (RNN).

The training data 65 define relations between vectors of target genomes (therapeutic drugs) and vectors of pluralities of subgenomes included in the target genomes. For example, a vector of a target genome corresponds to input data and a plurality of subgenomes serves as correct answer values of output data therefor.

The information processing apparatus executes training by error back propagation so that output upon input of a vector of a target genome into the trained model 70 approaches vectors of its subgenomes. The information processing apparatus adjusts parameters of the trained model 70 (executes machine training) by repeatedly executing the above described process on the basis of the relations included in the training data 65, the relations each being between: a vector of a target genome; and vectors of a plurality of subgenomes.

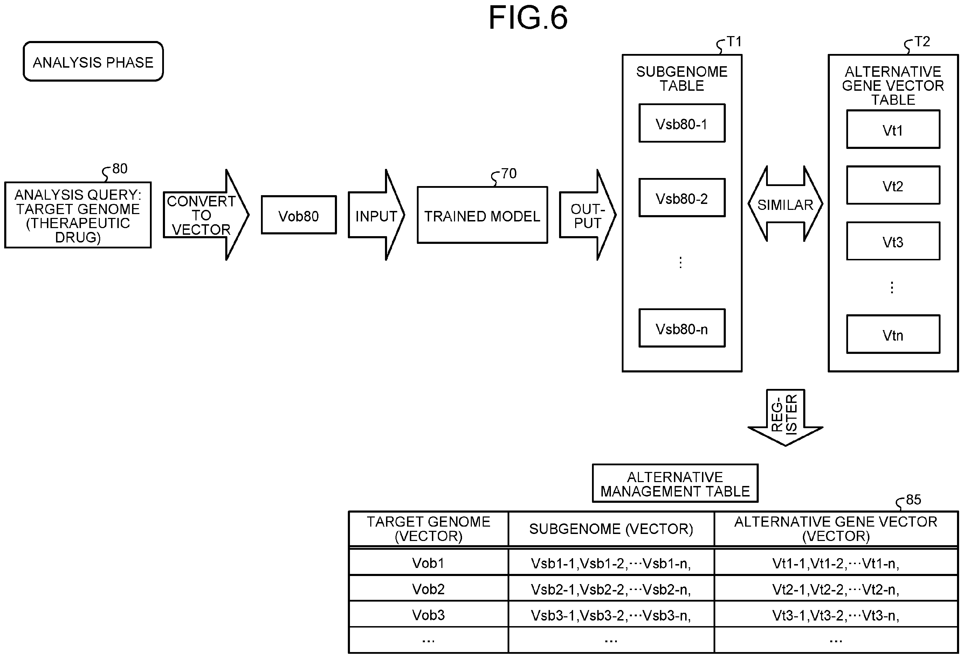

FIG. 6 is a diagram for explanation of an example of a process in an analysis phase of the information processing apparatus according to the embodiment. In the analysis phase, the information processing apparatus executes the following process by using the trained model 70 that has been trained in the training phase.

In response to the information processing apparatus receiving an analysis query 80 that specifies a target genome (a therapeutic drug), the information processing apparatus converts the target genome in the analysis query 80 to a vector Vob80. By inputting the vector Vob80 to the trained model 70, the information processing apparatus calculates a plurality of vectors (Vsb80-1, Vsb80-2, Vsb80-3, ..., Vsb80-n) corresponding to its subgenomes and stores the calculated plurality of vectors into a subgenome table T1.

The information processing apparatus makes a comparison among degrees of similarity between plural vectors (Vt1, Vt2, Vt3, ..., Vtn) corresponding respectively to alternative gene vectors stored in an alternative gene vector table T2 and the plurality of vectors (Vsb80-1, Vsb80-2, Vsb80-3, ..., Vsb80-n) to determine vectors of similar alternative gene vectors. The information processing apparatus registers the vector of the target genome, the vectors of the subgenomes, and the vectors of the similar alternative gene vectors, in association with one another, into an alternative management table 85.

As described above, the information processing apparatus according to the embodiment executes training of the trained model 70 beforehand, on the basis of the training data 65 defining the relations between the vectors of the target genomes and the vectors of their subgenomes. By inputting a vector of an analysis query into the trained model 70 that has been trained, the information processing apparatus calculates vectors of subgenomes corresponding to the target compound in the analysis query. Using the vectors of the subgenomes output from the trained model 70 facilitates detection of substitutable gene vectors that are gene vectors similar to the subgenomes included in the target genome.

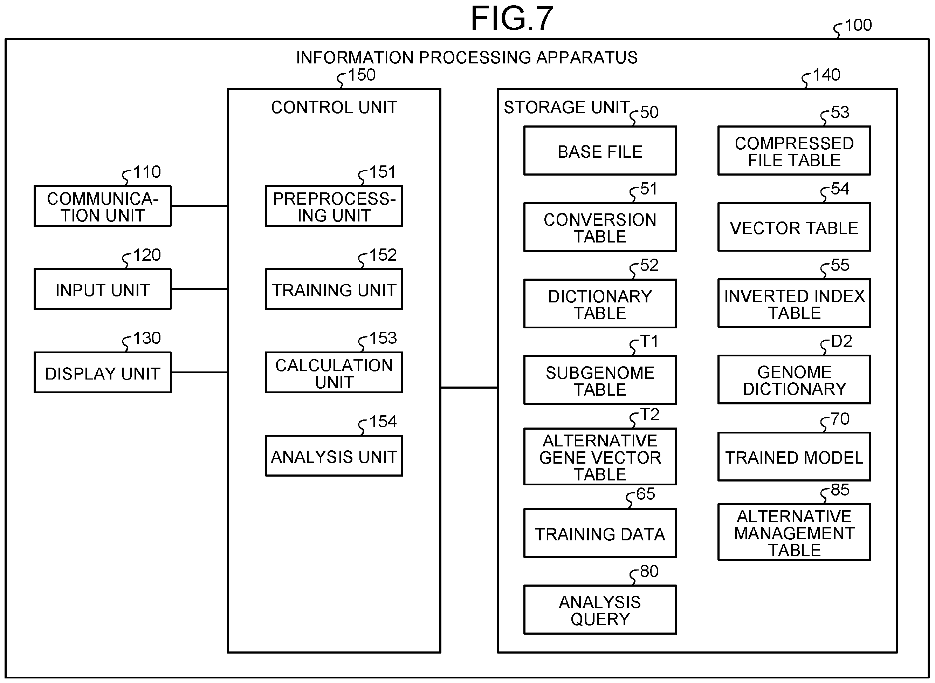

An example of a configuration of the information processing apparatus according to the first embodiment will be described next. FIG. 7 is a functional block diagram illustrating the configuration of the information processing apparatus according to the first embodiment. As illustrated in FIG. 7, this information processing apparatus 100 has a communication unit 110, an input unit 120, a display unit 130, a storage unit 140, and a control unit 150.

The communication unit 110 is connected to, for example, an external device by wire or wirelessly and transmits and receives information to and from, for example, the external device. For example, the communication unit 110 is implemented by a network interface card (NIC). The communication unit 110 may be connected to a network not illustrated in the drawings.

The input unit 120 is an input device that inputs various types of information to the information processing apparatus 100. The input unit 120 corresponds to, for example, a keyboard and a mouse, or a touch panel.

The display unit 130 is a display device that displays information output from the control unit 150. The display unit 130 corresponds to, for example, a liquid crystal display, an organic electro luminescence (EL) display, or a touch panel.

The storage unit 140 has a base file 50, a conversion table 51, a dictionary table 52, a compressed file table 53, a vector table 54, and an inverted index table 55. Furthermore, the storage unit 140 has the subgenome table T1, the alternative gene vector table T2, a genome dictionary D2, the training data 65, the trained model 70, the analysis query 80, and the alternative management table 85. The storage unit 140 is implemented by, for example: a semiconductor memory element, such as a random access memory (RAM) or a flash memory; or a storage device, such as a hard disk or an optical disk.

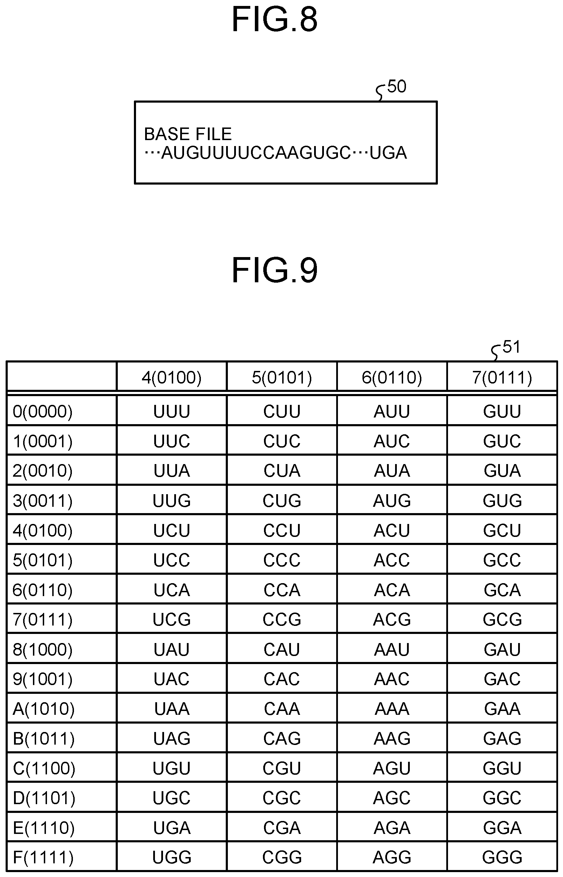

The base file 50 is a file that holds information including a sequence of plural bases. FIG. 8 is a diagram illustrating an example of a data structure of a base file. As illustrated in FIG. 8, the base file 50 is represented by four types of symbols, each of which is "A", "G", "C", "T", or "U".

The conversion table 51 is a table associating codons and codes of the codons with each other. A sequence of three bases is called a "codon". FIG. 9 is a diagram illustrating an example of a data structure of a conversion table. As illustrated in FIG. 9, codons are respectively associated with codes. For example, a codon, "UUU", has a code, "40h(01000000)". Herein, "h" indicates being a hexadecimal number.

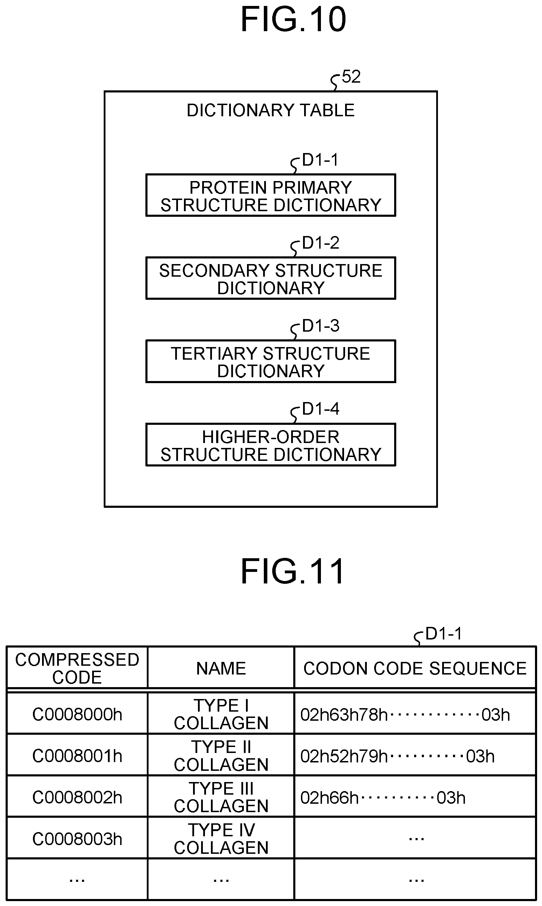

The dictionary table 52 is a table that holds various dictionaries. FIG. 10 is a diagram illustrating an example of a data structure of a dictionary table. As illustrated in FIG. 10, this dictionary table 52 has a protein primary structure dictionary D1-1, a secondary structure dictionary D1-2, a tertiary structure dictionary D1-3, and a higher-order structure dictionary D1-4.

The protein primary structure dictionary D1-1 is dictionary data defining relations between compressed codes of proteins and sequences of codons composing the proteins. FIG. 11 is a diagram illustrating an example of a data structure of a protein primary structure dictionary. As illustrated in FIG. 11, the protein primary structure dictionary D1-1 associates the compressed codes, names, and codon code sequences with one another. The compressed codes are compressed code sequences of codons (or symbol sequences of amino acids). The names are names of the proteins. The codon code sequences are sequences of compressed codes of the codons. Sequences of symbols of amino acids, instead of the codon code sequences, may be associated with the compressed codes of the protein primary structures.

For example, a compressed code, "C0008000h", is assigned to a protein primary structure, "type I collagen". A codon code sequence corresponding to the compressed code, "C0008000h", is "02h63h78h ... 03h".

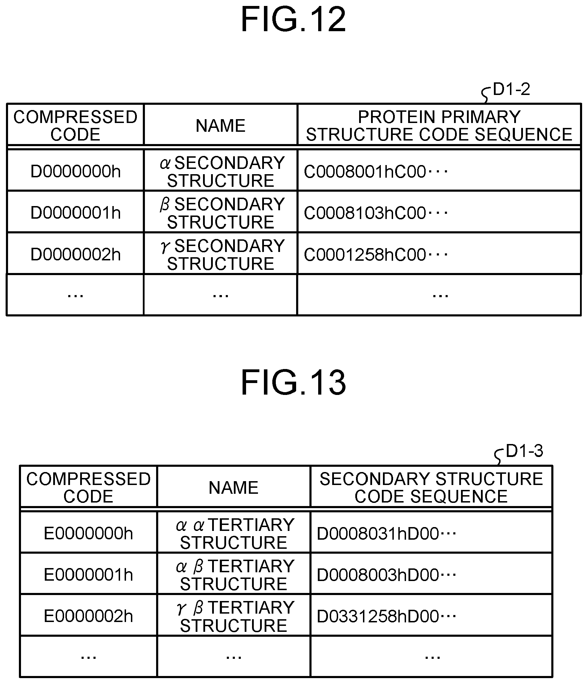

The secondary structure dictionary D1-2 is dictionary data defining relations between sequences of compressed codes of protein primary structures and compressed codes of secondary structures. FIG. 12 is a diagram illustrating an example of a data structure of a secondary structure dictionary. As illustrated in FIG. 12, the secondary structure dictionary D1-2 associates the compressed codes, names, and protein primary structure code sequences with one another. The compressed codes are compressed codes assigned to secondary structures of proteins. The names are names of the secondary structures. The protein primary structure code sequences are sequences of compressed codes of protein primary structures corresponding to the secondary structures.

For example, a compressed code, "D0000000h", is assigned to a secondary structure, "α secondary structure". A protein primary structure code sequence corresponding to the compressed code, "D0000000h", is "C0008001hC00 ...".

The tertiary structure dictionary D1-3 is dictionary data defining relations between sequences of compressed codes of secondary structures and compressed codes of tertiary structures. FIG. 13 is a diagram illustrating an example of a data structure of a tertiary structure dictionary. As illustrated in FIG. 13, the tertiary structure dictionary D1-3 associates the compressed codes, names, and secondary structure code sequences with one another. The compressed codes are compressed codes that have been assigned to the tertiary structures. The names are names of the tertiary structures. The secondary structure code sequences are sequences of compressed codes of secondary structures corresponding to the tertiary structures.

For example, a compressed code, "E0000000h", is assigned to a tertiary structure, "αα tertiary structure". A secondary structure code sequence corresponding to the compressed code, "E0000000h", is "D0008031hD00 ...".

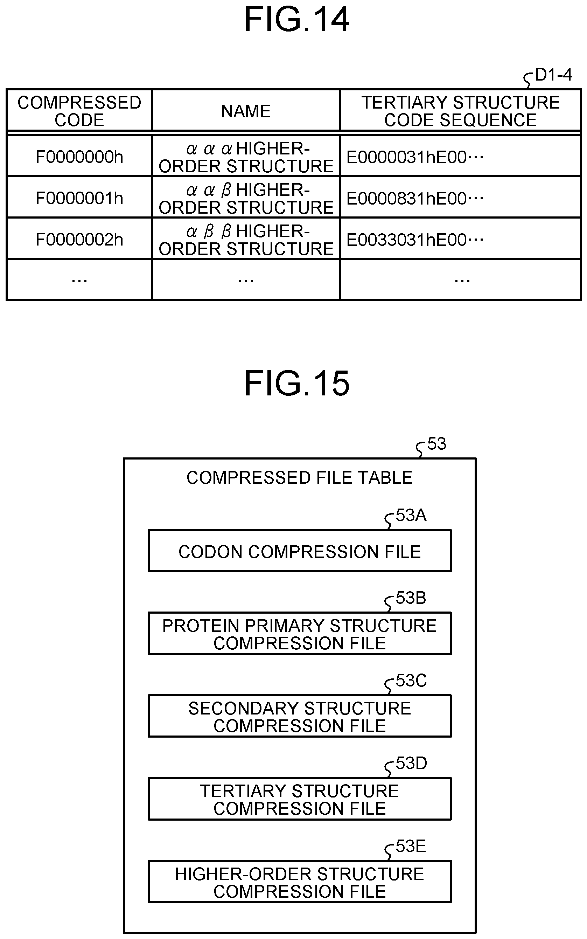

The higher-order structure dictionary D1-4 is dictionary data defining relations between sequences of compressed codes of tertiary structures and compressed codes of higher-order structures. FIG. 14 is a diagram illustrating an example of a data structure of a higher-order structure dictionary. As illustrated in FIG. 14, the higher-order structure dictionary D1-4 associates the compressed codes, names, and tertiary structure code sequences with one another. The compressed codes are compressed codes that have been assigned to the higher-order structures. The names are names of the higher-order structures. The tertiary structure code sequences are sequences of compressed codes of tertiary structures corresponding to the higher-order structures.

For example, a compressed code, "F0000000h", is assigned to a higher-order structure, "ααα higher-order structure". A tertiary structure code sequence corresponding to the compressed code, "F0000000h" is "E0000031hE00 ...".

The description of FIG. 7 will now be resumed. The compressed file table 53 is a table that holds various compressed files. FIG. 15 is a diagram illustrating an example of a data structure of a compressed file table. As illustrated in FIG. 15, this compressed file table 53 has a codon compression file 53A, a protein primary structure compression file 53B, a secondary structure compression file 53C, a tertiary structure compression file 53D, and a higher-order structure compression file 53E.

The codon compression file 53A is a file having the bases compressed in units of codons, the bases being included in the base file 50.

The protein primary structure compression file 53B is a file having the sequences coded in units of protein primary structures, the sequences being of the compressed codes of the codons included in the codon compression file 53A.

The secondary structure compression file 53C is a file having the sequences coded in units of secondary structures, the sequences being of the compressed codes of the protein primary structures included in the protein primary structure compression file 53B.

The tertiary structure compression file 53D is a file having the sequences coded in units of tertiary structures, the sequences being of the compressed codes of the secondary structures included in the secondary structure compression file 53C.

The higher-order structure compression file 53E is a file having the sequences coded in units of higher-order structures, the sequences being of the compressed codes of the tertiary structures included in the tertiary structure compression file 53D.

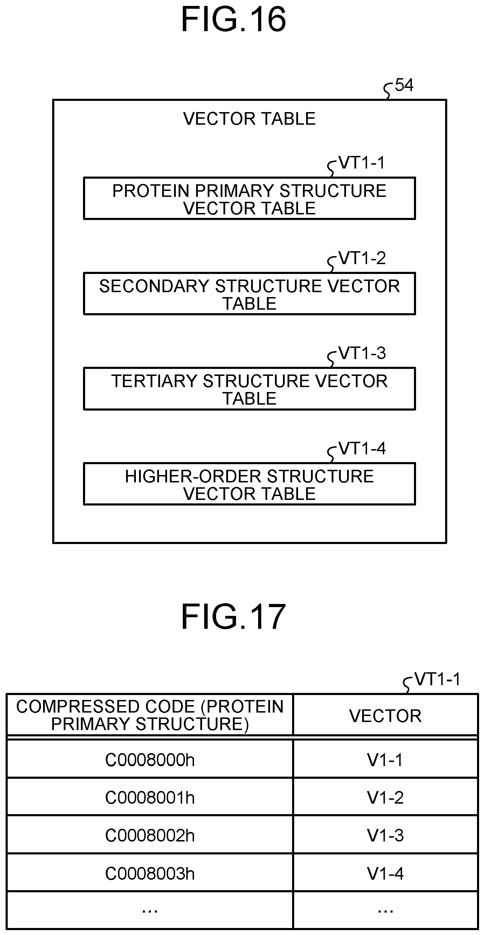

The vector table 54 is a table that holds vectors corresponding to protein primary structures, secondary structures, tertiary structures, and higher-order structures. FIG. 16 is a diagram illustrating an example of a data structure of a vector table. As illustrated in FIG. 16, this vector table 54 has a protein primary structure vector table VT1-1, a secondary structure vector table VT1-2, a tertiary structure vector table VT1-3, and a higher-order structure vector table VT1-4.

The protein primary structure vector table VT1-1 is a table that holds vectors corresponding to protein primary structures. FIG. 17 is a diagram illustrating an example of a data structure of a protein primary structure vector table. As illustrated in FIG. 17, the protein primary structure vector table VT1-1 has compressed codes of the protein primary structures, and the vectors that have been assigned to the compressed codes of these protein primary structures, in association with each other. The vectors of the protein primary structures are calculated by Poincare Embeddings. Poincare Embeddings will be described later.

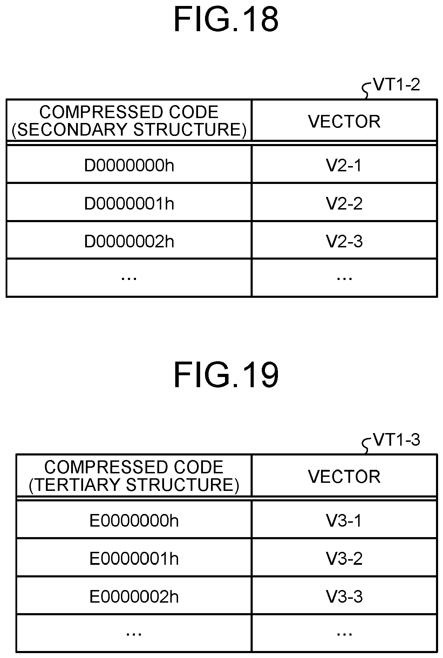

The secondary structure vector table VT1-2 is a table that holds vectors corresponding to secondary structures. FIG. 18 is a diagram illustrating an example of a data structure of a secondary structure vector table. As illustrated in FIG. 18, the secondary structure vector table VT1-2 has compressed codes of the secondary structures and the vectors that have been assigned to the compressed codes of the secondary structures, in association with each other. The vectors of the secondary structures are each calculated by adding up the vectors of the protein primary structures included in that secondary structure.

The tertiary structure vector table VT1-3 is a table that holds vectors corresponding to tertiary structures. FIG. 19 is a diagram illustrating an example of a data structure of a tertiary structure vector table. As illustrated in FIG. 19, the tertiary structure vector table VT1-3 has compressed codes of the tertiary structures and the vectors that have been assigned to the compressed codes of the tertiary structures, in association with each other. The vectors of the tertiary structures are each calculated by adding up the vectors of the secondary structures included in that tertiary structure.

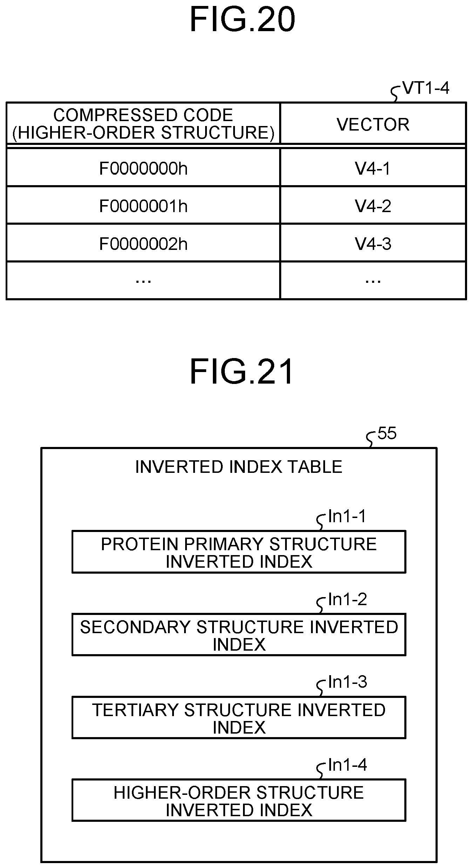

The higher-order structure vector table VT1-4 is a table that holds vectors corresponding to higher-order structures. FIG. 20 is a diagram illustrating an example of a data structure of a higher-order structure vector table. As illustrated in FIG. 20, the higher-order structure vector table VT1-4 has compressed codes of the higher-order structures and the vectors that have been assigned to the compressed codes of the higher-order structures, in association with each other. The vectors of the higher-order structures are each calculated by adding up the vectors of the tertiary structures included in that higher-order structure.

The description of FIG. 7 will now be resumed. The inverted index table 55 is a table that holds various inverted indices. FIG. 21 is a diagram illustrating an example of a data structure of an inverted index table. As illustrated in FIG. 21, the inverted index table 55 has a protein primary structure inverted index In1-1, a secondary structure inverted index In1-2, a tertiary structure inverted index In1-3, and a higher-order structure inverted index In1-4.

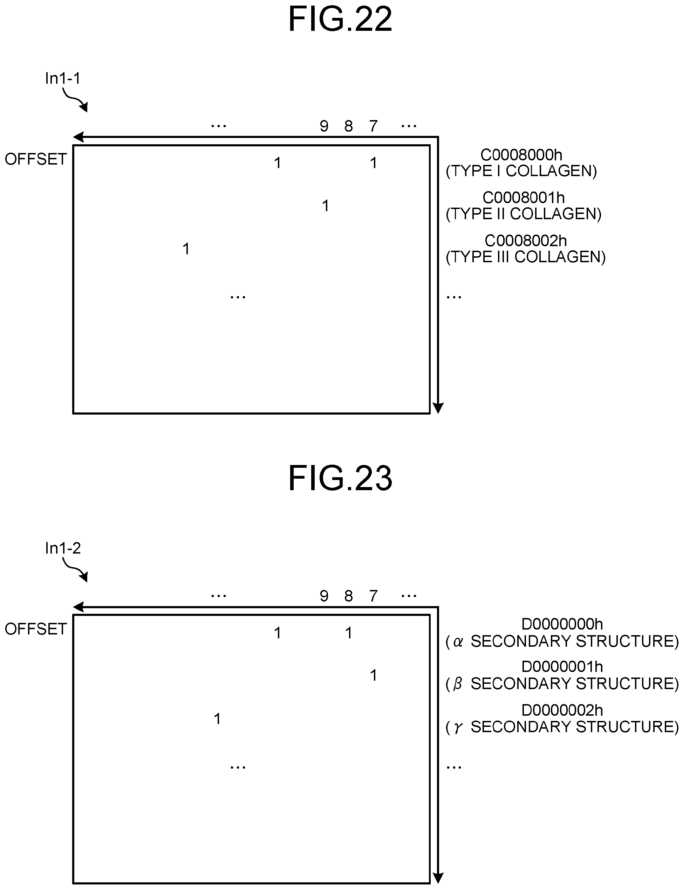

FIG. 22 is a diagram illustrating an example of a data structure of a protein primary structure inverted index. The horizontal axis of the protein primary structure inverted index In1-1 is an axis corresponding to offsets. The vertical axis of the protein primary structure inverted index In1-1 is an axis corresponding to compressed codes of protein primary structures. The protein primary structure inverted index In1-1 is represented by a bitmap of "0" or "1" and the whole bitmap is set to "0" in the initial state.

For example, the compressed code of the protein primary structure at the head of the protein primary structure compression file 53B has an offset of "0". In a case where the code, "C0008000h (type I collagen)", of a protein primary structure is included at the eighth position from the head of the protein primary structure compression file 53B, the bit at a position where the column of the offset of "7" in the protein inverted index In1-1 and the row of the code, "C0008000h (type I collagen)", of the protein intersect each other is "1".

FIG. 23 is a diagram illustrating an example of a data structure of a secondary structure inverted index. The horizontal axis of the secondary structure inverted index In1-2 is an axis corresponding to offsets. The vertical axis of the secondary structure inverted index In1-2 is an axis corresponding to compressed codes of secondary structures. The secondary structure inverted index In1-2 is represented by a bitmap of "0" or "1" and the whole bitmap is set to "0" in the initial state.

For example, the compressed code of the secondary structure at the head of the secondary structure compression file 53C has an offset of "0". In a case where the code, "D000000h (α secondary structure)", of a secondary structure is included at the eighth position from the head of the secondary structure compression file 53C, the bit at a position where the column of the offset of "7" in the secondary structure inverted index In1-2 and the row of the compressed code, "D0000000h (α secondary structure)", of the secondary structure intersect each other is "1".

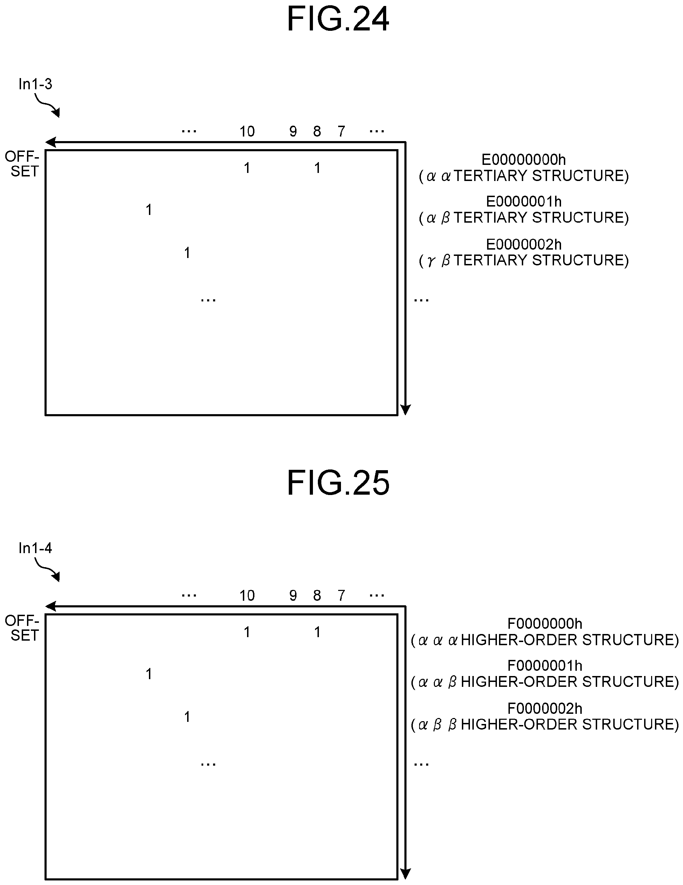

FIG. 24 is a diagram illustrating an example of a data structure of a tertiary structure inverted index. The horizontal axis of the tertiary structure inverted index In1-3 is an axis corresponding to offsets. The vertical axis of the tertiary structure inverted index In1-3 is an axis corresponding to compressed codes of tertiary structures. The tertiary structure inverted index In1-3 is represented by a bitmap of "0" or "1" and the whole bitmap is set to "0" in the initial state.

For example, the compressed code of the tertiary structure at the head of the tertiary structure compression file 53D has an offset of "0". In a case where the code, "E0000000h (αα tertiary structure)", of a tertiary structure is included at the eleventh position from the head of the tertiary structure compression file 53D, the bit at a position where the column of the offset of "10" in the tertiary structure inverted index In1-3 and the row of the compressed code, "E0000000h (αα tertiary structure)", of the tertiary structure intersect each other is "1".

FIG. 25 is a diagram illustrating an example of a data structure of a higher-order structure inverted index. It is the diagram illustrating the example of the data structure of the higher-order structure inverted index. The horizontal axis of the higher-order structure inverted index In1-4 is an axis corresponding to offsets. The vertical axis of the higher-order structure inverted index In1-4 is an axis corresponding to compressed codes of higher-order structures. The higher-order structure inverted index In1-4 is represented by a bitmap of "0" or "1" and the whole bitmap is set to "0" in the initial state.

For example, the compressed code of the higher-order structure at the head of the higher-order structure compression file 53E has an offset of "0". In a case where the code, "F0000000h (ααα higher-order structure)", of a higher-order structure is included at the eleventh position from the head of the higher-order structure compression file 53E, the bit at a position where the column of the offset of "10" in the higher-order structure inverted index In1-4 and the row of the compressed code, "F0000000h (ααα higher-order structure)", of the higher-order structure intersect each other is "1".

The description of FIG. 7 will now be resumed. The alternative gene vector table T2 holds vectors of plural gene vectors. The gene vectors correspond to secondary structures of proteins. For example, the vectors stored in the alternative gene vector table T2 may be the vectors that have been registered in the secondary structure vector table VT1-2. A data structure of the alternative gene vector table T2, as described by reference to FIG. 6, has the vectors of the plural alternative gene vectors stored therein.

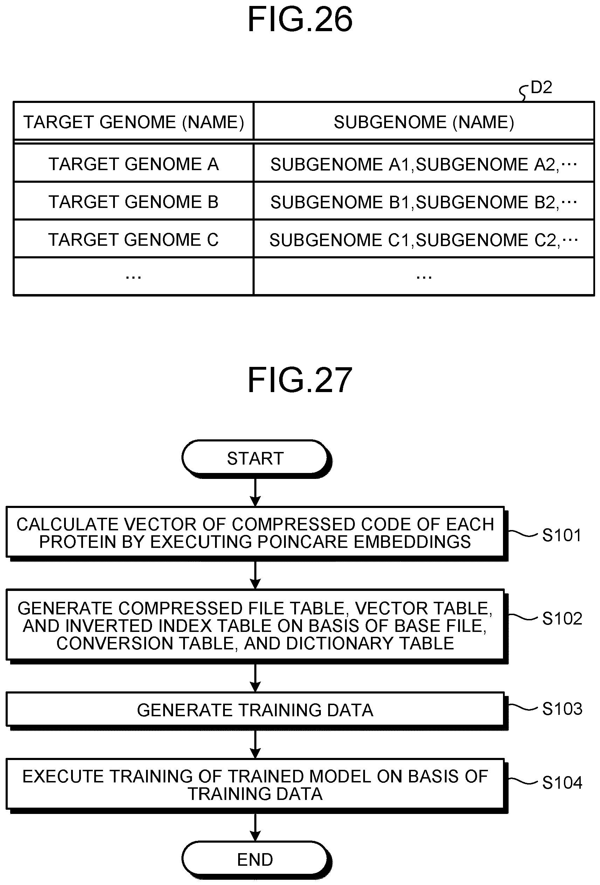

The genome dictionary D2 defines relations between names of target genomes and names of subgenomes included in these target genomes. FIG. 26 is a diagram illustrating an example of a data structure of a genome dictionary. As illustrated in FIG. 26, this genome dictionary D2 associates the names of the target vectors and the names of the pluralities of subgenomes with each other.

The training data 65 define relations each between a vector of a target genome and vectors of a plurality of subgenomes included in that target genome. The training data 65 have a data structure corresponding to the data structure of the training data described by reference to FIG. 5.

The trained model 70 is a model corresponding to, for example, a CNN or an RNN, and parameters are set for the trained model 70.

The analysis query 80 includes information on a target genome (therapeutic drug) to be analyzed. For example, the information on a target genome includes information on a base sequence corresponding to a higher-order structure.

The alternative management table 85 is a table holding vectors of subgenomes included in target genomes and vectors of gene vectors similar to these subgenomes in association with each other, the gene vectors being able to substitute for the subgenomes.

The control unit 150 has a preprocessing unit 151, a training unit 152, a calculation unit 153, and an analysis unit 154. The control unit 150 is implemented by, for example, a central processing unit (CPU) or a micro processing unit (MPU). Furthermore, the control unit 150 may be implemented by, for example, an integrated circuit, such as an application specific integrated circuit (ASIC) or a field programmable gate array (FPGA).

By executing various processes described below, the preprocessing unit 151 calculates a vector of a higher-order structure or tertiary structure corresponding to a target genome (therapeutic drug) and vectors of secondary structures corresponding to subgenomes, for example.

Firstly, the preprocessing unit 151 executes a process of generating the codon compression file 53A, a process of generating the protein primary structure compression file 53B, and a process of generating the protein primary structure vector table VT1-1 and the protein primary structure inverted index In1-1.

The preprocessing unit 151 compares the base file 50 and the conversion table 51 with each other, assigns compressed codes, in units of codons, to the base sequence in the base file 50, and generates the codon compression file 53A.

The preprocessing unit 151 compares the codon compression file 53A and the protein primary structure dictionary D1-1 with each other, assigns compressed codes, in units of protein primary structures, to sequences of the compressed codes of the codons included in the codon compression file 53A, and generates the protein primary compression file 53B.

In response to the preprocessing unit 151 generating the protein primary structure compression file 53B, the preprocessing unit 151 calculates vectors of the protein primary structures (the compressed codes of the protein primary structures) by embedding the compressed codes of the protein primary structures in Poincare space. A process of calculating a vector by embedding into Poincare space is a technique called Poincare Embeddings. For Poincare Embeddings, for example, a technique described in Non-Patent Literature by Valentin Khrulkov1 et al., "Hyperbolic Image Embeddings", published by Cornell University on April 3, 2019 may be used.

Poincare Embeddings are characterized in that a vector is assigned according to the embedded position in Poincare space and the more similar pieces of information are, the nearer their embedded positions are. Therefore, groups having similar characteristics are embedded at positions that are near one another in Poincare space and similar vectors are thus assigned to these groups. Although illustration will be omitted, the preprocessing unit 151 refers to a protein primary structure similarity table defining protein primary structures that are similar to each other, embeds a compressed code of each protein primary structure into Poincare space, and calculates a vector of the compressed code of each protein primary structure. The preprocessing unit 151 may execute Poincare Embeddings beforehand for a compressed code of each protein primary structure defined in the protein dictionary primary structure D1-1.

The preprocessing unit 151 generates the protein primary structure vector table VT1-1 by associating protein primary structures (compressed codes of protein primary structures) and vectors of the protein primary structures with each other. The preprocessing unit 151 generates the protein primary structure inverted index In1-1 on the basis of relations between the vectors of the protein primary structures and positions of the protein primary structures (the compressed codes of the protein primary structures) in the protein primary structure compression file 53B.

Subsequently, the preprocessing unit 151 executes a process of generating the secondary structure compression file 53C and a process of generating the secondary structure vector table VT1-2 and the secondary structure inverted index In1-2.

The preprocessing unit 151 compares the protein primary structure compression file 53B and the secondary structure dictionary D1-2 with each other, assigns compressed codes, in units of secondary structures, to sequences of the compressed codes of the protein primary structures included in the protein primary structure compression file 53B and generates the secondary structure compression file 53C.

The preprocessing unit 151 refers to the secondary structure dictionary D1-2 and determines protein primary structure code sequences (sequences of compressed codes of protein primary structures) corresponding to a compressed code of a secondary structure. The preprocessing unit 151 obtains vectors of the determined compressed codes of the protein primary structures from the protein primary structure vector table VT1-1, adds up the obtained vectors, and thereby calculates a vector of the compressed code of the secondary structure. By repeatedly executing the above described process, the preprocessing unit 151 calculates vectors of secondary structures.

By associating the secondary structures (the compressed codes of the secondary structures) and the vectors of the secondary structures with each other, the preprocessing unit 151 generates the secondary structure vector table VT1-2. The preprocessing unit 151 generates the secondary structure inverted index In1-2 on the basis of relations between the vectors of the secondary structures and positions of the secondary structures (the compressed codes of the secondary structures) in the secondary structure compression file 53C.

Subsequently, the preprocessing unit 151 executes a process of generating the tertiary structure compression file 53D and a process of generating the tertiary structure vector table VT1-3 and the tertiary structure inverted index In1-3.

The preprocessing unit 151 compares the secondary structure compression file 53C and the tertiary structure dictionary D1-3 with each other, assigns compressed codes, in units of tertiary structures, to sequences of the compressed codes of the secondary structures included in the secondary structure compression file 53C.

The preprocessing unit 151 refers to the tertiary structure dictionary D1-3 and determines secondary structure code sequences (sequences of compressed codes of secondary structures) corresponding to a compressed code of a tertiary structure. The preprocessing unit 151 obtains vectors of the determined compressed codes of the secondary structures from the secondary structure vector table VT1-2, adds up the obtained vectors, and thereby calculates a vector of the compressed code of the tertiary structure. By repeatedly executing the above described process, the preprocessing unit 151 calculates vectors of tertiary structures.

By associating the tertiary structures (the compressed codes of the tertiary structures) and the vectors of the tertiary structures with each other, the preprocessing unit 151 generates the tertiary structure vector table VT1-3. The preprocessing unit 151 generates the tertiary structure inverted index In1-3 on the basis of relations between the vectors of the tertiary structures and positions of the tertiary structures (the compressed codes of the tertiary structures) in the tertiary structure compression file 53D.

Subsequently, the preprocessing unit 151 executes a process of generating the higher-order structure compression file 53E and a process of generating the higher-order structure vector table VT1-4 and the higher-order structure inverted index In1-4.

The preprocessing unit 151 compares the tertiary structure compression file 53D and the higher-order structure dictionary D1-4 with each other, assigns compressed codes, in units of higher-order structures, to sequences of the compressed codes of the tertiary structures included in the tertiary structure compression file 53D, and generates the higher-order structure compression file 53E.

The preprocessing unit 151 refers to the higher-order structure dictionary D1-4 and determines tertiary structure code sequences (sequences of compressed codes of tertiary structures) corresponding to a compressed code of a higher-order structure. The preprocessing unit 151 obtains vectors of the determined compressed codes of the tertiary structures from the tertiary structure vector table VT1-3, adds up the obtained vectors, and thereby calculates a vector of the compressed code of the higher-order structure. By repeatedly executing the above described process, the preprocessing unit 151 calculates vectors of higher-order structures.

The preprocessing unit 151 generates the higher-order structure vector table VT1-4 by associating the higher-order structures (the compressed codes of the higher-order structures) and the vectors of the higher-order structures with each other. The preprocessing unit 151 generates the higher-order structure inverted index In1-4 on the basis of relations between the vectors of the higher-order structures and positions of the higher-order structures (the compressed codes of the higher-order structures) in the higher-order structure compression file 53E.

The following description is on an example of a process in which the preprocessing unit 151 generates the alternative gene vector table T2. For example, the preprocessing unit 151 sets, as is, the vectors of the tertiary structures included in the secondary structure vector table VT1-2, in the alternative gene vector table T2. In a case where the preprocessing unit 151 has received specification of a vector via the input unit 120, the preprocessing unit 151 may set the specified vector in the alternative gene genome table T2.

The following description is on an example of a process in which the preprocessing unit 151 generates the training data 65. The preprocessing unit 151 determines a relation between a name of a target genome and names of subgenomes, on the basis of the genome dictionary D2. The preprocessing unit 151 determines a vector of the target genome on the basis of the higher-order structure dictionary D1-4 and the higher-order structure vector table VT1-4, or the tertiary structure dictionary D1-4 and the tertiary structure vector table VT1-3, and the name of the target genome. The preprocessing unit 151 determines vectors of the subgenomes on the basis of the secondary structure dictionary D1-2 and secondary structure vector table VT1-2 and the names of the subgenomes. The preprocessing unit 151 determines the relation between the target genome and the subgenomes through this process and registers the relation into the training data 65.

The preprocessing unit 151 generates the training data 65 by repeatedly executing the above described process. The information processing apparatus 100 may obtain and use the training data 65 that have been generated beforehand, from, for example, an external device.

The description of FIG. 7 will now be resumed. The training unit 152 executes training of the trained model 70 by using the training data 65. A process by the training unit 152 corresponds to the process described by reference to FIG. 5. The training unit 152 obtains, from the training data 65, a pair of: a vector of a target genome (a therapeutic drug); vectors of subgenomes corresponding to the vector of this target genome. The training unit 152 adjusts parameters of the trained model 70 by executing training by error back propagation so that the value of output from the trained model 70 in a case where the vector of the target genome is input to the trained model 70 approaches the values of the vectors of the subgenomes.

The training unit 152 executes training of the trained model 70 by repeatedly executing the above described process for pairs of vectors of target genomes and vectors of subgenomes in the training data 65.

In a case where the calculation unit 153 has received specification by the analysis query 80, the calculation unit 153 calculates vectors of subgenomes included in the target genome in the analysis query 80, by using the trained model 70 that has been trained. A process by the calculation unit 153 corresponds to the process described by reference to FIG. 6. The calculation unit 153 may receive the analysis query 80 from the input unit 120 or may receive the analysis query 80 from an external device via the communication unit 110.

The calculation unit 153 obtains a base sequence of the target genome included in the analysis query 80. The calculation unit 153 compares the base sequence of the target genome and the conversion table 51 with each other, determines codons included in the base sequence of the target genome, and converts the base sequence of the target genome into compressed codes, in units of codons. Furthermore, the calculation unit 153 compares the codon code sequences compressed in units of codons and the protein primary structure dictionary D1-1 with each other and converts the codon code sequences into compressed codes, in units of protein primary structures.

The calculation unit 153 compares the converted compressed codes of the protein primary structures with the protein primary structure vector table VT1-1 and determines vectors of the compressed codes of the protein primary structures. By adding up the determined vectors of the compressed codes of the protein primary structures, the calculation unit 153 calculates the vector Vob80 corresponding to the target genome included in the analysis query 80.

In a case where a target genome has been specified by secondary structures of plural subgenomes, the calculation unit 153 executes the following process. The calculation unit 153 compares the secondary structures of the subgenomes of the target genome with the secondary structure dictionary D1-2 and secondary structure vector table VT1-2, and determines vectors of the secondary structures of the subgenomes included in the target genome. By adding up the determined vectors of the secondary structures of the subgenomes, the calculation unit 153 calculates a vector of the target genome.

The calculation unit 153 calculates plural vectors corresponding to the subgenomes by inputting the vector Vob80 into the trained model 70. The calculation unit 153 outputs the calculated vectors of the subgenomes, to the analysis unit 154. In the description hereinafter, the vectors of the subgenomes calculated by the calculation unit 153 will be respectively referred to as "analysis vectors". The calculation unit 153 stores the vectors (analysis vectors) of the subgenomes into the subgenome table T1.

The analysis unit 154 makes a search for information on alternative gene vectors having vectors similar to the analysis vectors, on the basis of the analysis vectors. On the basis of a result of the search, the analysis unit 154 registers the vectors of the subgenomes included in the target genome and the vectors (similar vectors described hereinafter) of the alternative gene vectors similar thereto, in association with each other, into the alternative management table 85.

For example, the analysis unit 154 calculates distances between an analysis vector and the vectors included in the alternative gene vector table T2 to determine any vector having a distance less than a threshold, the distance being from the analysis vector. Any vector included in the alternative gene vector table T2 and having the distance from the analysis vector is a "similar vector", the distance being less than the threshold. A gene vector corresponding to this similar vector is a substitutable gene vector.

The analysis unit 154 may determine a compressed code of the gene vector corresponding to the similar vector, on the basis of the secondary structure vector table VT1-2 and determine a protein primary structure included in the gene vector, on the basis of the determined compressed code of the gene vector, the secondary structure dictionary D1-2, and the protein primary structure dictionary D1-1. By executing this process, the analysis unit 154 makes a search for characteristics of the substitutable gene vector corresponding to the similar vector and registers the characteristics into the alternative management table 85. The characteristics of the substitutable gene vector are the protein included in the gene vector and the primary structure of the protein.

By repeatedly executing the above described process for the analysis vectors, the analysis unit 154 may make a search, for each of the analysis vectors, for characteristics of the gene vector corresponding to the similar vector and register the characteristics into the alternative management table 85. The analysis unit 154 may output the alternative management table 85 to the display unit 130 to cause the display unit 130 to display the alternative management table 85 or may transmit the alternative management table 85 to an external device connected to a network.

An example of a procedure by the information processing apparatus 100 according to the embodiment will be described next. FIG. 27 is a first flowchart illustrating a procedure by the information processing apparatus according to the embodiment. As illustrated in FIG. 27, the preprocessing unit 151 of the information processing apparatus 100 calculates vectors of compressed codes of proteins by executing Poincare Embeddings (Step S101) .

On the basis of the base file 50, the conversion table 51, and the dictionary table 52, the preprocessing unit 151 generates the compressed file table 53, the vector table 54, and the inverted index table 55 (Step S102) .

The preprocessing unit 151 generates the training data 65 (Step S103). On the basis of the training data 65, the training unit 152 of the information processing apparatus 100 executes training of the trained model 70 (Step S104) .

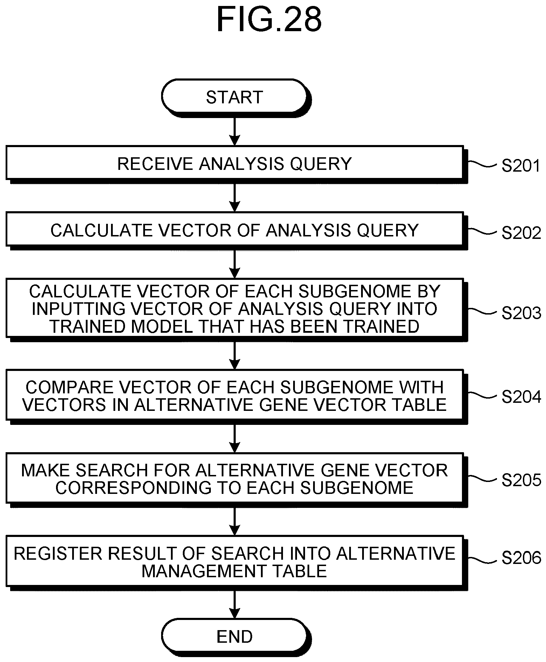

FIG. 28 is a second flowchart illustrating a procedure by the information processing apparatus according to the embodiment. The calculation unit 153 of the information processing apparatus 100 receives the analysis query 80 (Step S201). The calculation unit 153 calculates a vector of the analysis query 80 (target genome) (Step S202) .

The calculation unit 153 calculates vectors of subgenomes by inputting the calculated vector of the analysis query 80 into the trained model 70 that has been trained (Step S203). The analysis unit 154 of the information processing apparatus 100 compares the vectors of the subgenomes and the alternative gene vector table T2 with each other (Step S204).

The analysis unit 154 makes a search for substitutable gene vectors corresponding to the subgenomes (Step S205). The analysis unit 154 registers a result of the search into the alternative management table 85 (Step S206) .

Effects of the information processing apparatus 100 according to the embodiment will be described next. In the training phase, the information processing apparatus 100 executes training of the trained model 70 beforehand, on the basis of the training data 65 defining relations between vectors of target compounds (therapeutic drugs) and vectors of subgenomes. In the analysis phase, the information processing apparatus 100 calculates vectors of subgenomes corresponding to an analysis query (a target genome) by inputting the vector of the analysis query into the trained model 70 that has been trained. Using the vectors of the subgenomes output from the trained model 70 facilitates detection of substitutable gene vectors similar to the subgenomes included in the target genome.

For example, in a case where a subgenome included in a target genome is a rare subgenome, executing the process by the information processing apparatus 100 facilitates the search for an inexpensive gene vector serving as an alternative to that subgenome.

In the above described embodiment, the comparison is made at granularity of subgenomes (secondary structures) for a search to be made for a substitutable gene vector, but the embodiment is not limited to this example. For example, the information processing apparatus 100 may make a comparison at granularity of plural primary structures composing a subgenome for a search to be made for alternative primary structures.

### Second Embodiment

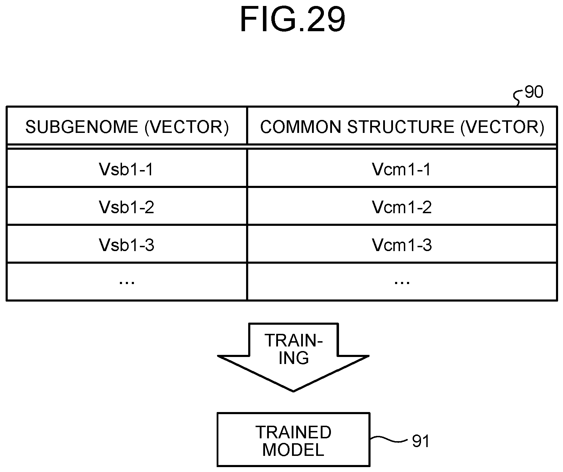

A second embodiment will be described next. FIG. 29 is a diagram for explanation of an example of a process in a training phase of an information processing apparatus according to the second embodiment. As illustrated in FIG. 29, by using training data 90, the information processing apparatus executes training of a trained model 91. The trained model 91 corresponds to, for example, a CNN or an RNN.

The training data 90 define relations between vectors of pluralities of subgenomes for synthesis of target genomes (therapeutic drugs) and vectors of common structures maintained in genetic modification based on gene vectors. For example, vectors of subgenomes correspond to input data, and vectors of plural common structures serve as correct answer values.

The information processing apparatus executes training by error back propagation, so that output upon input of a vector of a subgenome to the trained model 91 approaches the vector of each common structure. The information processing apparatus adjusts parameters of the trained model 91 (executes machine training) by repeatedly executing the above described process on the basis of the relations between the vectors of the subgenomes included in the training data 90 and the vectors of the common structures.

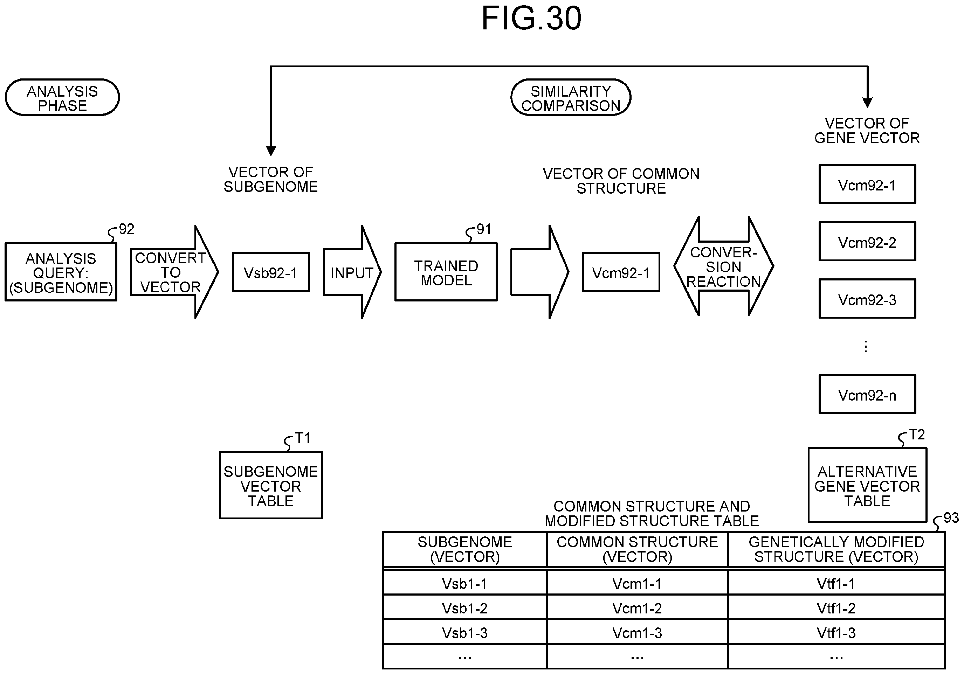

FIG. 30 is a diagram for explanation of a process by the information processing apparatus according to the second embodiment. Similarly to the information processing apparatus 100 of the first embodiment, the information processing apparatus according to the second embodiment may train the trained model 91 beforehand. Furthermore, as described already by reference to FIG. 29, the information processing apparatus trains the trained model 91 that is different from the trained model 70. The trained model 91 outputs a vector of a common structure in a case where a vector of an analysis query (subgenome) 92 is input to the trained model 91.

In response to the information processing apparatus receiving the analysis query 92 specifying the subgenome, the information processing apparatus converts the subgenome in the analysis query 92 into a vector Vsb92-1 by using a subgenome vector table T1. By inputting the vector Vsb92-1 of the subgenome into the trained model 91, the information processing apparatus calculates a vector Vcm92-1 corresponding to its common structure.

The information processing apparatus compares the vector Vsb92-1 of the subgenome and vectors of plural gene vectors included in an alternative gene vector table T2 with each other. The alternative gene vector table T2 corresponds to the alternative gene vector table T2 described with respect to the first embodiment.

The information processing apparatus determines a vector of a gene vector similar to the vector Vsb92-1 of the subgenome. For example, a vector Vt92-1 is determined as the vector of the gene vector similar to the vector Vsb92-1 of the subgenome. A vector of a common structure common to the subgenome having the vector Vsb92-1 and the gene vector having the vector Vt92-1 is then found to be the vector Vcm92-1 output from the trained model 91. Furthermore, a result of subtraction of the vector Vcm92-1 of the common structure from the vector Vt92-1 of the gene vector is a vector of a "genetically modified structure" corresponding to difference between the similar gene vector and the subgenome.

The information processing apparatus registers the relation between the vector of the common structure and the vector of the genetically modified structure into a common structure and genetically modified structure table 93. By repeatedly executing the above described process for vectors of subgenomes, the information processing apparatus generates the common structure and genetically modified structure table 93.

As described above, the information processing apparatus according to the second embodiment inputs the vector of the analysis query 92 into the trained model 91 that has been trained to calculate a vector of each common structure corresponding to the subgenome in the analysis query. Furthermore, by subtraction of a vector of a common structure from each of vectors of gene vectors similar to a subgenome, a vector of a genetically modified structure corresponding to difference between the similar subgenome and the gene vector is calculated. Using the above described vector of the common structure and vector of the genetically modified structure facilitates analysis for a better gene vector usable for synthesis and manufacture of the target genome.

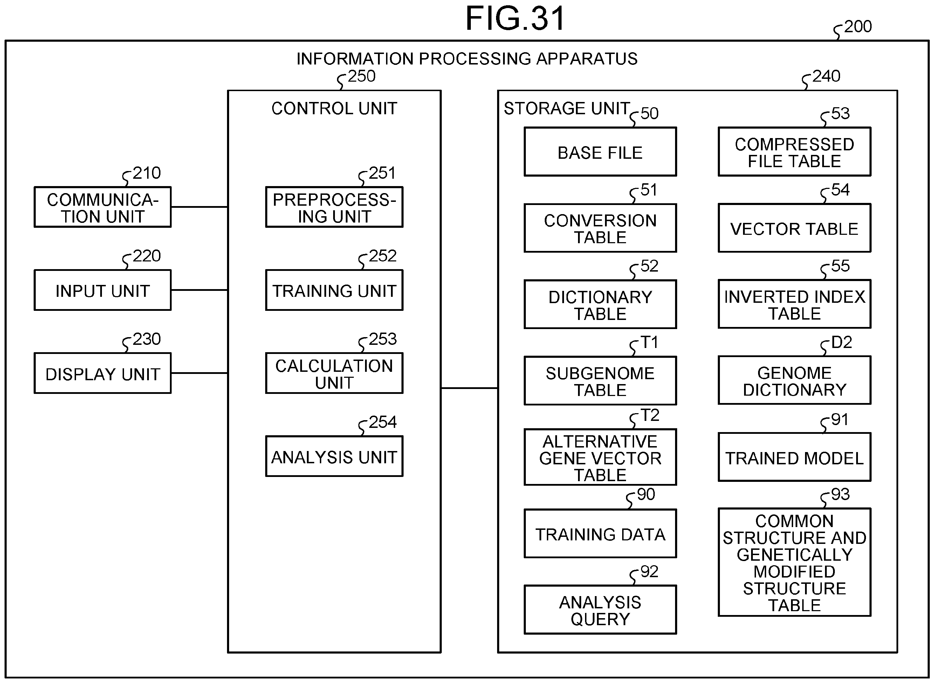

An example of a configuration of the information processing apparatus according to the second embodiment will be described next. FIG. 31 is a functional block diagram illustrating a configuration of the information processing apparatus according to the second embodiment. As illustrated in FIG. 31, this information processing apparatus 200 has a communication unit 210, an input unit 220, a display unit 230, a storage unit 240, and a control unit 250.

Description related to the communication unit 210, the input unit 220, and the display unit 230 is similar to the description related to the communication unit 110, the input unit 120, and the display unit 130 described with respect to the first embodiment.

The storage unit 240 has a base file 50, a conversion table 51, a dictionary table 52, a compressed file table 53, a vector table 54, and an inverted index table 55. Furthermore, the storage unit 240 has the subgenome table T1, the alternative gene vector table T2, a genome dictionary D2, the training data 90, the trained model 91, the analysis query 92, and the common structure and genetically modified structure table 93. The storage unit 240 is implemented by, for example: a semiconductor memory element, such as a random access memory (RAM) or a flash memory; or a storage device, such as a hard disk or an optical disk.

Description related to the base file 50, the conversion table 51, the dictionary table 52, the compressed file table 53, the vector table 54, the inverted index table 55, the subgenome table T1, the alternative gene vector table T2, and the genome dictionary D2 is similar to what has been described with respect to the first embodiment. The training data 90 are similar to those described by reference to FIG. 29. Description related to the trained model 91 and the analysis query 92 is similar to what has been described by reference to FIG. 30.

As described by reference to FIG. 30, the common structure and genetically modified structure table 93 includes information on genetically modified structure vectors for genetic modification from gene vectors similar to common structure vectors to subgenomes. For example, the common structure and genetically modified structure table 93 in FIG. 30 includes genetically modified structure vectors corresponding to Vcm92-1. A vector obtained by adding up vectors of common structures and vectors of genetically modified structures is a vector corresponding to a vector of a gene vector.

The description of FIG. 31 will now be resumed. The control unit 250 has a preprocessing unit 251, a training unit 252, a calculation unit 253, and an analysis unit 254. The control unit 250 is implemented by, for example, a CPU or an MPU. Furthermore, the control unit 250 may be implemented by, for example, an integrated circuit, such as an ASIC or FPGA.

Description related to the preprocessing unit 251 is similar to the description of the process related to the preprocessing unit 151 described with respect to the first embodiment. The base file 50, the conversion table 51, the dictionary table 52, the compressed file table 53, the vector table 54, the inverted index table 55, the subgenome table T1, and the alternative gene vector table T2 are generated by the preprocessing unit 251. The preprocessing unit 251 may obtain the training data 90 from an external device or the preprocessing unit 251 may generate the training data 90.

In a case where the calculation unit 253 has received specification by the analysis query 92, the calculation unit 253 calculates a vector of each common structure to be subjected to a genetic modification via a synthetic pathway for the subgenome in the analysis query 92, by using the trained model 91 that has been trained. The calculation unit 253 outputs the calculated vector of each common structure, to the analysis unit 254.

In the description hereinafter, the vectors of common structures calculated by the calculation unit 253 will each be referred to as the "common structure vector".

The analysis unit 254 generates the common structure and genetically modified mechanism table 93 on the basis of the vector of the subgenome in the analysis query 92, the common structure vectors, and the gene vector table T2. An example of a process by the analysis unit 254 will be described hereinafter.

The analysis unit 254 calculates distances between a vector of a subgenome and vectors included in the alternative gene vector table T2 respectively to determine any vector having a distance less than a threshold, the distance being from the vector of the subgenome. Any vector included in the alternative gene vector table T2 and having a distance less than the threshold will be referred to as a "similar vector", the distance being from the vector of the subgenome.

By subtracting the common structure vector from the similar vector, the analysis unit 254 calculates a vector of a genetically modified structure, and determines a correspondence relation between the common structure vector and the vector of the genetically modified structure. The analysis unit 254 registers the common structure vector and the vector of the genetically modified structure into the common structure and genetically modified structure table 93. By repeatedly executing the above described process, the analysis unit 254generates the common structure and genetically modified structure table 93. The analysis unit 245 may output the common structure and genetically modified structure table 93 to the display unit 230 to cause the display unit 230 to display the common structure and genetically modified structure table 93, or may transmit the common structure and genetically modified structure table 93 to an external device connected to a network.

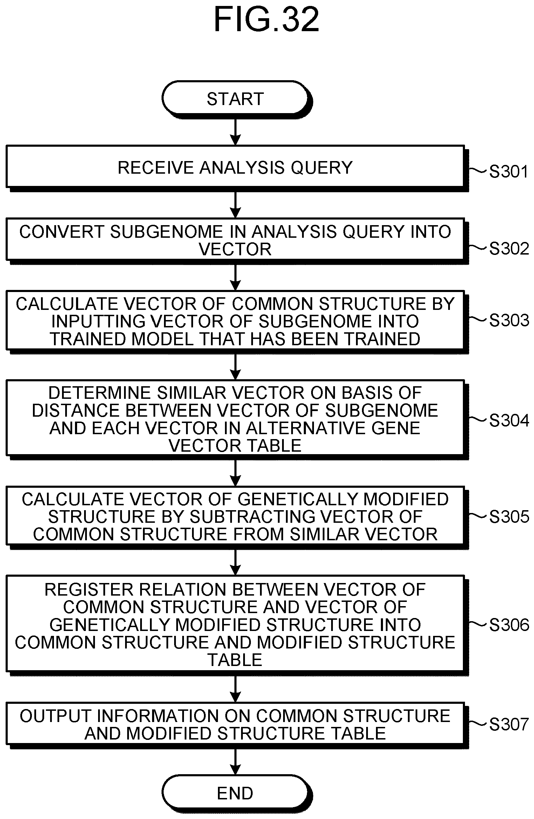

An example of a procedure by the information processing apparatus 200 according to the second embodiment will be described next. FIG. 32 is a flowchart illustrating a procedure by the information processing apparatus according to the second embodiment. The calculation unit 253 of the information processing apparatus 200 receives the analysis query 92 (Step S301).

On the basis of the subgenome table T1, the calculation unit 253 converts the subgenome in the analysis query 92 into a vector (Step S302).

By inputting the vector of the subgenome into the trained model 91 that has been trained, the calculation unit 253 calculates a vector of a common structure (Step S303). On the basis of distances between the vector of the common structure and vectors in the alternative gene vector table T2, the analysis unit 254 of the information processing apparatus 200 determines any similar vector (Step S304).

The analysis unit 254 calculates a vector of a genetically modified structure by subtracting the vector of the common structure from the vector of each gene vector similar to the subgenome (Step S305). The analysis unit 254 registers a relation between the vector of the common structure and the vector of the genetically modified structure into the common structure and genetically modified structure table 93 (Step S306). The analysis unit 254 outputs information on the common structure and genetically modified structure table (Step S307).

Effects of the information processing apparatus 200 according to the second embodiment will be described next. The information processing apparatus 200 inputs a vector of the analysis query 92, into the trained model 91 that has been trained to calculate a vector of each common structure corresponding to the subgenome in the analysis query. Furthermore, by subtraction of a vector of a common structure from the vector of each gene vector similar to the subgenome, a vector of a genetically modified structure corresponding to difference between the similar subgenome and the gene vector is calculated. Using the above described vector of the common structure and vector of the genetically modified structure facilitates analysis for a better gene vector usable for genetic modification to the target genome and resynthesis and manufacture of the target genome.

Subgenomes and gene vectors are each a secondary structure composed of plural protein primary structures. Furthermore, using dispersion vectors of the protein primary structures enables estimation of protein primary structures adjacent to a certain protein primary structure and is able to be applied to evaluation of bonding and stability of each protein primary structure. With respect to genetic modification from a gene vector to a proven subgenome, executing machine training on the basis of dispersion vectors of plural protein primary structures composing a secondary structure of the subgenome or gene vector enables improvement of: application of the gene vector; the genetic modification; and accuracy of analysis of resynthesis.

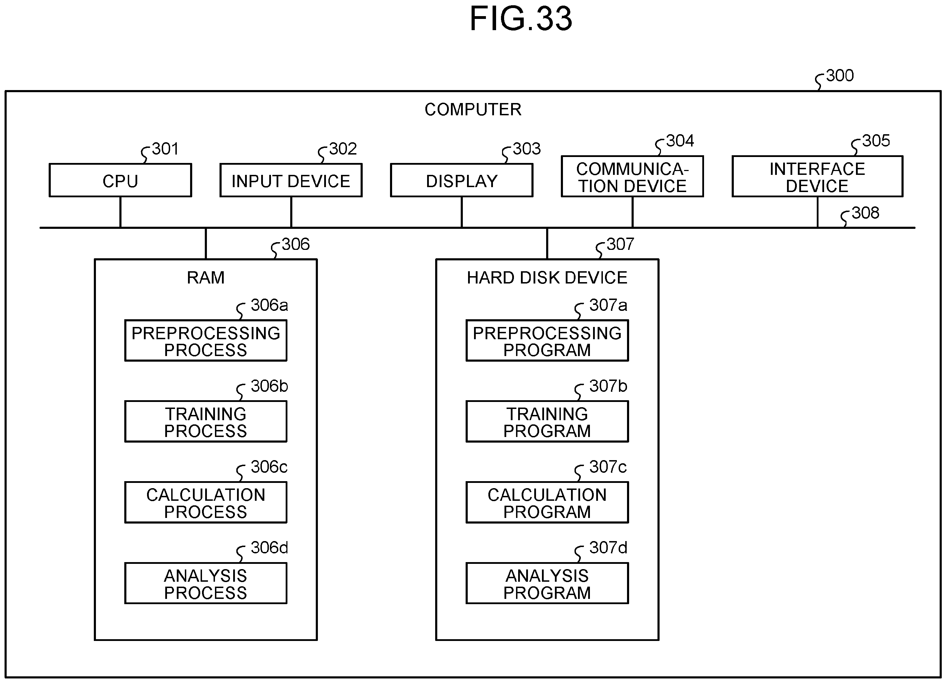

An example of a hardware configuration of a computer that implements functions that are the same as those of the above described information processing apparatus 100 (200) according to the embodiment will be described next. FIG. 33 is a diagram illustrating an example of a hardware configuration of a computer that implements functions that are the same as those of the information processing apparatus according to the embodiment.

As illustrated in FIG. 9, a computer 300 has a CPU 301 that executes various types of arithmetic processing, an input device 302 that receives data input from a user, and a display 303. Furthermore, the computer 300 has: a communication device 304 that transfers data to and from, for example, an external device, via a wired or wireless network; and an interface device 305. The computer 300 also has a RAM 306 that temporarily stores therein various types of information, and a hard disk device 307. Each of these devices 301 to 307 is connected to a bus 308.

The hard disk device 307 has a preprocessing program 307a, a training program 307b, a calculation program 307c, and an analysis program 307d. Furthermore, the CPU 301 reads the programs 307a to 307d and load the read programs 307a to 307d into the RAM 306.

The preprocessing program 307a functions as a preprocessing process 306a. The training program 307b functions as a training process 306b. The calculation program 307c functions as a calculation process 306c. The analysis program 307d functions as an analysis process 306d.

A process by the preprocessing process 306a corresponds to the process by the preprocessing unit 151 or 251. A process by the training process 306b corresponds to the process by the training unit 152 or 252. A process by the calculation process 306c corresponds to the process by the calculation unit 153 or 253. A process by the analysis process 306d corresponds to the process by the analysis unit 154.

The programs 307a to 307d are not necessarily stored in the hard disk device 307 beforehand. For example, each program is stored in a "portable physical medium", such as a flexible disk (FD), a CD-ROM, a DVD, a magnetooptical disk, or an IC card, which is to be inserted in the computer 300. The computer 300 may then read and execute the programs 307a to 307d therefrom.

### Reference Signs List

110, 210 COMMUNICATION UNIT
120, 220 INPUT UNIT
130, 230 DISPLAY UNIT
140, 240 STORAGE UNIT
150, 250 CONTROL UNIT
151, 251 PREPROCESSING UNIT
152, 252 TRAINING UNIT
153, 253 CALCULATION UNIT
154, 254 ANALYSIS UNIT

## Claims

1. An information processing program that causes a computer to execute a process comprising:
executing training of a trained model on the basis of training data defining relations between vectors corresponding to genomes and vectors respectively corresponding to pluralities of subgenomes composing the genomes; and
in a case where a genome to be analyzed has been received, calculating vectors of a plurality of subgenomes corresponding to the genome to be analyzed by inputting the genome to be analyzed into the trained model.

2. The information processing program according to claim 1, wherein the process further includes making a search for alternative gene vectors that are able to substitute for the subgenomes, on the basis of degrees of similarity between the calculated vectors of the plurality of subgenomes and vectors of a plurality of alternative gene vectors that are candidates for alternatives.

3. The information processing program according to claim 1, wherein the genome to be analyzed includes a plurality of secondary structures of a protein and the process further includes calculating a vector of the genome to be analyzed, by adding up vectors of the plurality of secondary structures included in the genome to be analyzed.

4. An information processing program that causes a computer to execute a process comprising:
executing training of a trained model on the basis of training data having, in association with each other, stored vectors of a plurality of subgenomes included in a synthetic pathway for manufacture of a genome and stored vectors of common structures representing structures common to structures of the subgenomes and structures of gene vectors; and
in a case where input of a subgenome to be analyzed has been received, calculating a vector of a common structure corresponding to the subgenome to be analyzed, by inputting a vector of the subgenome to be analyzed into the trained model.

5. The information processing program according to claim 4, wherein the process further includes: making a search for a vector of a gene vector similar to the vector of the subgenome on the basis of similarity relations between the vector of the subgenome and vectors of a plurality of gene vectors that are candidates for an alternative; and calculating a vector of a genetically modified structure representing a structure of a portion corresponding to difference between a structure of the subgenome and a structure of the gene vector obtained by the search, on the basis of the vector obtained by the search and the calculated vector of the common structure.

6. An information processing method comprising:
executing training of a trained model on the basis of training data defining relations between vectors corresponding to genomes and vectors respectively corresponding to pluralities of subgenomes composing the genomes; and
in a case where a genome that is a target to be analyzed has been received, inputting the genome that is the target to be analyzed into the trained model and calculating vectors of a plurality of subvectors corresponding to a gene vector of the target to be analyzed.

7. The information processing method according to claim 6, further including making a search, on the basis of degrees of similarity between the calculated vectors of a plurality of subgenomes and vectors of a plurality of alternative gene vectors that are candidates for alternatives, the search being for alternative gene vectors that are able to substitute for the subgenomes.

8. The information processing method according to claim 6, wherein the genome that is the target to be analyzed includes a plurality of secondary structures of a protein and the information processing method further includes calculating a vector of the genome that is the target to be analyzed, by adding up vectors of the plurality of secondary structures included in the genome that is the target to be analyzed.

9. An information processing method comprising:
executing training of a trained model on the basis of training data defining relations between vectors of a plurality of subgenomes included in a synthetic pathway for manufacture of a genome and vectors of common structures representing structures common to structures of the subgenomes and structures of gene vectors; and
in a case where a subgenome to be analyzed has been received, calculating a vector of a common structure corresponding to the subgenome to be analyzed, by inputting a vector of the subgenome to be analyzed into the trained model.

10. The information processing method according to claim 9, further including: making a search for a vector of a gene vector similar to the vector of the subgenome on the basis of degrees of similarity between the vector of the subgenome and vectors of a plurality of gene vectors serving as candidates for alternatives; and calculating a vector of a genetically modified structure representing a structure of a portion corresponding to difference between a structure of the subgenome and a structure of the gene vector obtained by the search, on the basis of the vector obtained by the search and the calculated vector of the common structure.

11. An information processing apparatus, comprising a control unit configured to:
execute training of a trained model on the basis of training data defining relations between vectors corresponding to genomes and vectors respectively corresponding to pluralities of subgenomes composing the genomes;
input, in a case where a genome to be analyzed has been received, the genome to be analyzed into the trained model; and
calculate vectors of a plurality of subgenomes corresponding to the genome to be analyzed.

12. The information processing apparatus according to claim 11, wherein the control unit is further configured to make a search for alternative gene vectors that are able to substitute for the subgenomes, on the basis of degrees of similarity between the vectors of the calculated plurality of subgenomes and vectors of a plurality of alternative gene vectors that are candidates for alternatives.

13. The information processing apparatus according to claim 11, wherein the genome to be analyzed includes a plurality of secondary structures of a protein, and the control unit is further configured to calculate a vector of the genome to be analyzed by adding up vectors of the plurality of secondary structures included in the genome to be analyzed.
